(19) Europäisches Patentamt
European Patent Office
Office européen des brevets

(11) EP 1 321 131 A2

(12) **EUROPÄISCHE PATENTANMELDUNG**

(43) Veröffentlichungstag:
**25.06.2003 Patentblatt 2003/26**

(51) Int Cl.[7]: **A61K 7/13**, D06P 3/00

(21) Anmeldenummer: **02027905.5**

(22) Anmeldetag: **13.12.2002**

(84) Benannte Vertragsstaaten:
**AT BE BG CH CY CZ DE DK EE ES FI FR GB GR IE IT LI LU MC NL PT SE SI SK TR**
Benannte Erstreckungsstaaten:
**AL LT LV MK RO**

(30) Priorität: **22.12.2001 DE 10163803**

(71) Anmelder: **Henkel Kommanditgesellschaft auf Aktien**
**40589 Düsseldorf-Holthausen (DE)**

(72) Erfinder:
- **Ehlert, Manuela**
**51379 Leverkusen (DE)**
- **Höffkes, Horst, Dr.**
**40595 Düsseldorf (DE)**
- **Hollenberg, Detlef, Dr.**
**40699 Erkrath (DE)**

(54) **Farberhaltung**

(57) Der Gegenstand der vorliegenden Erfindung betrifft die Verwendung von farbstabilisierendem Wirkstoff oder Wirkstoffgemischen zur Verbesserung der Waschechtheit von Färbungen keratinischer Fasern, umfassend als Wirkstoff wenigstens ein N-haltiges Silikon und/oder Polystyrolsulfonat und/oder Pyrrolidon, wobei sich die Waschechtheit gefärbter Fasern, insbesondere keratinischer Fasern, gegebenenfalls durch Verwendung eines zusätzlichen Polymers noch verbessern läßt.

EP 1 321 131 A2

## Beschreibung

Die Erfindung betrifft die Verwendung von N-haltigen Silikonen, Polystyrolsulfonaten und/oder Pyrrolidonen zur Verbesserung der Waschechtheit von Färbungen keratinischer Fasern, entsprechende Zubereitungen sowie Verfahren zum Färben von Fasern.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle. Sieht man von den Blondiermitteln, die eine oxidative Aufhellung der Haare durch Abbau der natürlichen Haarfarbstoffe bewirken, ab, so sind im Bereich der Haarfärbung im wesentlichen drei Typen von Haarfärbemitteln von Bedeutung:

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muß aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet. Weisen die im Verlauf der Farbausbildung gebildeten bzw. direkt eingesetzten Farbstoffe deutlich unterschiedliche Echtheiten (z. B. UV-Stabilität, Schweißechtheit, Waschechtheit etc.) auf, so kann es mit der Zeit zu einer erkennbaren und daher unerwünschten Farbverschiebung kommen.

Dieses Phänomen tritt verstärkt auf, wenn die Frisur Haare oder Haarzonen unterschiedlichen Schädigungsgrades aufweist. Ein Beispiel dafür sind lange Haare, bei denen die lange Zeit allen möglichen Umwelteinflüssen ausgesetzten Haarspitzen in der Regel deutlich stärker geschädigt sind als die relativ frisch nachgewachsenen Haarzonen.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt.

Schließlich hat in jüngster Zeit ein neuartiges Färbeverfahren große Beachtung gefunden. Bei diesem Verfahren werden Vorstufen des natürlichen Haarfarbstoffes Melanin auf das Haar aufgebracht; diese bilden dann im Rahmen oxidativer Prozesse im Haar naturanaloge Farbstoffe aus. Ein solches Verfahren mit 5,6-Dihydroxyindolin als Farbstoffvorprodukt wurde in der EP-B1-530 229 beschrieben. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so daß auf keine weiteren Oxidationsmittel zurückgegriffen werden muß. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden. Auch hier können dann Probleme hinsichtlich der Echtheit der Färbungen auftreten.

Es hat nicht an Anstrengungen gefehlt, die Echtheit von Färbungen keratinischer Fasern zu verbessern. Eine Entwicklungsrichtung ist die Optimierung der Farbstoffe selbst bzw. die Synthese neuer, modifizierter Farbstoffmoleküle. Eine weitere Entwicklungsrichtung ist die Suche nach Zusätzen für die Färbemittel, um die Echtheit der Färbungen zu erhöhen. Eine bekannte Problemlösung ist, dem Färbemittel UV-Filter zuzusetzen. Diese Filtersubstanzen werden beim Färbeprozeß zusammen mit dem Farbstoff auf das Haar aufgebracht, wodurch in vielen Fällen eine deutliche Steigerung der Stabilität der Färbung gegen die Einwirkung von Tages- oder Kunstlicht erzielt wird.

Aufgabe der vorliegenden Erfindung ist es, die Nachteile des Standes der Technik zu überwinden und insbesondere ein Verfahren zur Verfügung zu stellen mittels dessen eine deutliche Steigerung der Stabilität der Färbung keratinischer Fasern gegenüber Umwelteinflüssen, wie Wasser, Tages- oder Kunstlicht oder dergleichen erreichbar ist.

Es wurde nun überraschenderweise gefunden, daß durch die Verwendung von N-haltigen Silikonen und/oder Polystyrolsulfonaten und/oder Pyrrolidonen die Waschechtheit von Färbungen insbesondere keratinischer Fasern signifikant gesteigert werden kann. Unter Waschechtheit im Sinne der Erfindung ist die Erhaltung der ursprünglichen Färbung hinsichtlich Nuance und/oder Intensität zu verstehen, wenn die keratinische Faser dem wiederholten Einfluß von wäßrigen Mitteln, insbesondere tensidhaltigen Mitteln wie Shampoos, ausgesetzt wird.

Ein erster Gegenstand der vorliegenden Erfindung ist die Verwendung eines farbstabilisierendem Wirkstoffes oder Wirkstoffgemisches (A), umfassend als Wirkstoff wenigstens ein N-haltiges Silikon, Polystyrolsulfonat und/oder

Pyrrolidon, zur Verbesserung der Waschechtheit von Färbungen von Fasern, insbesondere von keratinischen Fasern.

Der erfindungsgemäß verwendete Wirkstoff oder Wirkstoffgemisch (A) verbessert die Waschechtheit von Färbungen auf künstlichen Fasern wie Polyestern und natürlichen Fasern wie Baumwolle und insbesondere keratinischen Fasern.

Unter keratinischen Fasern werden erfindungsgemäß Pelze, Wolle, Federn und insbesondere menschliche Haare verstanden.

Das N-haltige Silikon als erfindungsgemäßer Wirkstoff (A1) kann vorzugsweise ausgewählt werden aus der Gruppe umfassend Siloxanpolymere mit wenigstens einer Aminogruppe, Siloxanpolymere mit wenigstens einer endständigen Aminogruppe, Amodimethicon, Trimethylsilylamodimethicone, und/oder Aminoethylaminopropylsiloxan-Dimethylsiloxan-Copolymer.

Erfindungsgemäß bevorzugt ist die Verwendung eines Aminosiloxans entsprechend der nachstehenden allgemeinen Formel I,

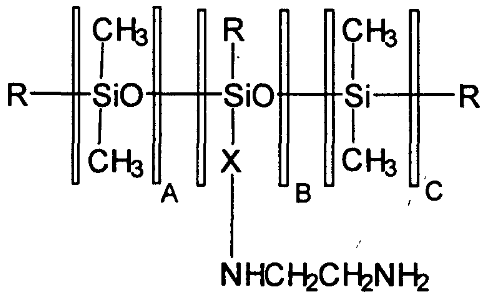

(I)

wobei R = OH oder $CH_3$; X = Alkylgruppe mit 1 bis 4 C-Atomen, vorzugsweise Propyl oder Isopropyl und A, B und C = Copolymereinheiten, die taktische und/oder ataktische Polymerblöcke ausbilden können, sind.

Erfindungsgemäß am meisten bevorzugt ist Amodimethicon, Amodimethicon haltige Emulsionen oder Fluide. Emulsionen, die erfindungsgemäß bevorzugt eingesetzt werden können sind Dow Corning® 949, hierbei handelt es sich um eine kationische Emulsion enthaltend Amodimethicon, Cetrimoniumchlorid und Trideceth-12; Dow Corning® 939, hierbei handelt es sich um eine Emulsion enthaltend Amodimethicon, Cetrimoniumchlorid und Trideceth-12; Dow Corning® 929, hierbei handelt es sich um eine kationische Emulsion enthaltend Amodimethicon, Talktrimoniumchlorid und Nonoxynol-10; Dow Corning® 7224 oder 1401, basierend auf Trimethylsilylamodimethicon, Octoxynol-40, Isolaureth-6 und Glycol; Dow Corning® 2-8194 Mikroemulsion (26%ig) auf Basis eines aminfunktionalisierten Siliconpolymers; Dow Corning® 2-8177 Mikroemulsion (12%ig) auf Basis eines aminfunktionalisierten Siliconpolymers; Dow Corning® 2-8566 Amino Fluid auf Basis eines aminfunktionalisierten Polydimethylsiloxans; erhältlich bei der Firma Dow Corning.

Das Silikon als erfindungsgemäßer Wirkstoff (A1) wird in einer Menge von 0,001 bis 20 Gew.% bezogen auf das gesamte Mittel, bevorzugt in Mengen von 0,001 bis 10 Gew.% und ganz besonders bevorzugt in Mengen von 0,001 bis 5 Gew.% verwendet.

Ein weiterer erfindungsgemäß verwendbarer Wirkstoff (A2) ist Polystyrolsulfonat. Die Molmassen von Polystyrolsulfonat machen vorzugsweise 300 bis 1000000 g/mol aus. Das Polystyrolsulfonat kann durch Copolymerisation mit geeigneten Comonomeren, wie Acrylnitril, Butadien oder durch Abmischen mit anderen Polymeren modifiziert werden.

Vorzugsweise kann Flexan® 130, hierbei handelt es sich um ein Polystyrolsulfonat, erhältlich von National Starch and Company, erfindungsgemäß verwendet werden.

Das Polystyrolsulfonat als erfindungsgemäßer Wirkstoff (A2) wird in einer Menge von 0,001 bis 20 Gew.% bezogen auf das gesamte Mittel, bevorzugt in Mengen von 0,001 bis 10 Gew.% und ganz besonders bevorzugt in Mengen von 0,001 bis 5 Gew.% verwendet.

Erfindungsgemäß verwendbare Pyrrolidone als Wirkstoff (A3) haben die nachstehende allgemeine Formel II,

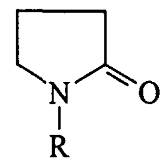

(II)

wobei R = Alkyl, Alkenyl, Hydroxyalkyl, Hydroxyalkenyl, Aminoalkyl, Aminoalkenyl, vorzugsweise mit 8 bis 30 C-Atomen, bevorzugt 8 bis 22 C-Atome, ist.

Das Pyrrolidon gemäß der allgemeinen Formel (II) kann auch in Form seines Salzes, vorzugsweise quarternisiert mit Dimethylsulfat (DMS) oder als Alkylhalogenid, verwendet werden.

Besonders geeignete Pyrrolidone können ausgewählt werden aus der Gruppe umfassend: N-Laurylpyrrolidon, wie Surfadone® LP-300 (ISP) und/oder N-Caprylylpyrrolidon, wie Surfadone® LP-100 (ISP) und/oder N-Methylpyrrolidon wie Flouwet® EA 093 (Clariant).

Das Pyrrolidonderivat als erfindungsgemäßer Wirkstoff (A3) wird in einer Menge von 0,001 bis 20 Gew.% bezogen auf das gesamte Mittel, bevorzugt in Mengen von 0,001 bis 10 Gew.% und ganz besonders bevorzugt in Mengen von 0,001 bis 5 Gew.% verwendet.

Erfindungsgemäß ist es auch möglich, die erfindungsgemäßen Wirkstoffe (A1 - A3) als Gemisch von mindestens zwei der Wirkstoffe zu verwenden. Das Mischungsverhältnis kann in diesem Falle von 1:99 bis 99:1 betragen. Bevorzugt werden als Wirkstoffgemisch die Silikone (A1) mit den Pyrrolidonen (A2) verwendet, wenngleich jede beliebige Kombination dieser Wirkstoffe erfindungsgemäß möglich ist. Die erfindungsgemäße Wirkstoffmischung (A) wird in einer Menge von 0,001 bis 20 Gew.% bezogen auf das gesamte Mittel, bevorzugt in Mengen von 0,001 bis 10 Gew. % und ganz besonders bevorzugt in Mengen von 0,001 bis 5 Gew.% verwendet.

Überraschenderweise wird die Wirkung des zuvor beschriebenen Wirkstoffes oder Wirkstoffgemisches (A) noch weiter verbessert, wenn zusätzlich Kohlenhydrate verwendet werden. Die erfindungsgemäße, synergistische Wirkung aus dem Wirkstoff und/oder Wirkstoffgemisch (A) mit den Kohlenhydraten (B) zeichnet sich insbesondere durch eine Restrukturierung vorbelasteter Haarfasern, sowie mehr Glanz, besserer Elastizität und einer erhöhten Reißfestigkeit sowie einer geringeren Splißrate als auch besseren Beständigkeit gegen äußere Einflüße auf das Haar, wie beispielsweise Föhnen aus. Insbesondere sind im Sinne der vorliegenden Erfindung unter Kohlenhydraten (B) zu verstehen:

- Kohlenhydrate, Zuckeralkohole und Zucker sowie deren Salze,
  - insbesondere Monosaccharide, Disaccharide, Trisaccharide und Oligosaccharide, wobei diese auch in Form von Aldosen, Ketosen und/oder Lactosen, sowie geschützt durch übliche und in der Literatur bekannte -OH - und -NH - Schutzgruppen, wie beispielsweise die Triflatgruppe, die Trimethylsilylgruppe oder Acylgruppen sowie weiterhin in Form der Methylether und als Phosphatester, vorliegen können,
  - Aminodesoxyzucker, Desoxyzucker, Thiozucker, wobei diese auch in Form von Aldosen, Ketosen und/oder Lactosen, sowie geschützt durch übliche und in der Literatur bekannte -OH - und -NH - Schutzgruppen, wie beispielsweise die Triflatgruppe, die Trimethylsilylgruppe oder Acylgruppen sowie weiterhin in Form der Methylether und als Phosphatester, vorliegen können,

Ganz besonders bevorzugt sind hierunter Monosaccharide mit 3 bis 8 C - Atomen, wie beispielsweise Triosen, Tetrosen, Pentosen, Hexosen, Heptosen und Octosen, wobei diese auch in Form von Aldosen, Ketosen und/oder Lactosen sowie geschützt durch übliche und in der Literatur bekannte -OH - und -NH - Schutzgruppen, wie beispielsweise die Triflatgruppe, die Trimethylsilylgruppe oder Acylgruppen sowie weiterhin in Form der Methylether und als Phosphatester, vorliegen können,

Weiterhin sind ganz besonders bevorzugt Oligosaccharide mit bis zu 50 Monomereinheiten, wobei diese auch in Form von Aldosen, Ketosen und/oder Lactosen sowie geschützt durch übliche und in der Literatur bekannte -OH - und -NH - Schutzgruppen, wie beispielsweise die Triflatgruppe, die Trimethylsilylgruppe oder Acylgruppen sowie weiterhin in Form der Methylether und als Phosphatester, vorliegen können.

Beispielhaft für die erfindungsgemäßen Kohlenhydrate seien erwähnt Sorbit, Inosit, Mannit, Tetrite, Pentite, Hexite, Threit, Erythrit, Adonit, Arabit, Xylit, Dulcit, Erythrose, Threose, Arabinose, Ribose, Xylose, Lyxose, Glucose, Galactose, Mannose, Allose, Altrose, Gulose, Idose, Talose, Fructose, Sorbose, Psicose, Tegatose, Desoxyribose,

Glucosamin, Galaktosamin, Rhamnose, Digitoxose, Thioglucose, Saccharose, Lactose, Trehalose, Maltose, Cellobiose, Melibiose, Gestiobiose, Rutinose, Raffinose sowie Cellotriose. Weiterhin sei auf die einschlägige Fachliteratur wie beispielsweise Beyer-Walter, Lehrbuch der organischen Chemie, S. Hirzel Verlag Stuttgart, 19. Auflage, Abschnitt III , Seiten 393 und folgende verwiesen.

Selbstverständlich umfaßt die erfindungsgemäße Lehre alle isomeren Formen, wie cis - trans - Isomere, Diastereomere, Epimere, Anomere und chirale Isomere.

Erfindungsgemäß ist es auch möglich, eine Mischung aus mehreren Kohlenhydraten einzusetzen.

Die erfindungsgemäßen Kohlehydrate sind in den Mitteln in Konzentrationen von 0,01 Gew.% bis zu 20 Gew. %, vorzugsweise von 0,05 Gew.% bis zu 15 Gew.% und ganz besonders bevorzugt in Mengen von 0,1 Gew.% bis zu 10 Gew.% enthalten.

Als weiterhin vorteilhaft hat es sich gezeigt, daß Polymere die farberhaltende Wirkung des erfindungsgemäßen Wirkstoffes unterstützen können. In einer weiteren erfindungsgemäß verwendbaren Ausrührungsform werden daher Polymere zugesetzt, wobei sich kationische, anionische und amphotere Polymere als besonders wirksam erwiesen haben.

Die erfindungsgemäß zusätzlich verwendbaren Polymere sind im Sinne dieser Erfindung nicht identisch mit den Wirkstoffen.

Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine $C_{1-4}$-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (III),

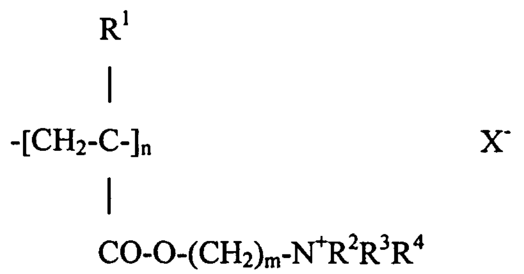

(III)

in der $R^1$ = -H oder $-CH_3$ ist, $R^2$, $R^3$ und $R^4$ unabhängig voneinander ausgewählt sind aus $C_{1-4}$-Alkyl-, -Alkenyl- oder -Hydroxyalkylgruppen, m = 1, 2, 3 oder 4, n eine natürliche Zahl und $X^-$ ein physiologisch verträgliches organisches oder anorganisches Anion ist, sowie Copolymere, bestehend im wesentlichen aus den in Formel (III) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:

- $R^1$ steht für eine Methylgruppe
- $R^2$, $R^3$ und $R^4$ stehen für Methylgruppen
- m hat den Wert 2.

Als physiologisch verträgliches Gegenionen $X^-$ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylen-bisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (III) enthalten als nichtionogene Monomereinheiten bevorzugt Acrylamid, Methacrylamid, Acrylsäure-$C_{1-4}$-alkylester und Methacrylsäure-$C_{1-4}$-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare® SC 92 erhältlich.

Weitere bevorzugte kationische Polymere sind beispielsweise

- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat® 50,
- kationische Guar-Derivate, wie insbesondere die unter den Handelsnamen Cosmedia®Guar und Jaguar® vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden.
- quaternierter Polyvinylalkohol,

sowie die unter den Bezeichnungen

- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind.

Erfindungsgemäß bevorzugte kationische Polymere sind kationische Cellulose-Derivate und Chitosan und dessen Derivate, insbesondere die Handelsprodukte Polymer®JR 400, Hydagen® HCMF und Kytamer® PC, kationische Guar-Derivate, kationische Honig-Derivate, insbesondere das Handelsprodukt Honeyquat® 50, kationische Alkylpolyglycodside gemäß der DE-PS 44 13 686 und Polymere vom Typ Polyquaternium-37.

Bei den anionischen Polymeren, welche die farberhaltende Wirkung des erfindungsgemäßen Wirkstoffes unterstützen können, handelt es sich um ein anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik®11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel®305 der Firma SEPPIC enthalten. Die Verwendung dieses Compounds, das neben der Polymerkomponente eine Kohlenwasserstoffmischung ($C_{13}$-$C_{14}$-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel®600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol® im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnungg Stabileze® QM im Handel erhältlich.

Weiterhin können als Polymere zur Steigerung der Wirkung des erfindungsgemäßen Wirkstoffes amphotere Polymere als Bestandteil eingesetzt. Unter dem Begriff amphotere Polymere werden sowohl solche Polymere, die im Molekül sowohl freie Aminogruppen als auch freie -COOH- oder $SO_3H$-Gruppen enthalten und zur Ausbildung innerer Salze befähigt sind, als auch zwitterionische Polymere, die im Molekül quartäre Ammoniumgruppen und $-COO^-$- oder $-SO_3^-$-Gruppen enthalten, und solche Polymere zusammengefaßt, die -COOH- oder $SO_3H$-Gruppen und quartäre Ammoniumgruppen enthalten.

Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Bevorzugt eingesetzte amphotere Polymere sind solche Polymerisate, die sich im wesentlichen zusammensetzen aus

(a) Monomeren mit quartären Ammoniumgruppen der allgemeinen Formel (II),

$$R^5\text{-CH=CR}^6\text{-CO-Z-}(C_nH_{2n})\text{-N}^{(+)}R^7R^8R^9\ A^{(-)} \qquad \text{(II)}$$

in der $R^5$ und $R^6$ unabhängig voneinander stehen für Wasserstoff oder eine Methylgruppe und $R^7$, $R^8$ und $R^9$ unabhängig voneinander für Alkylgruppen mit 1 bis 4 Kohlenstoff-Atomen, Z eine NH-Gruppe oder ein Sauerstoffatom, n eine ganze Zahl von 2 bis 5 und $A^{(-)}$ das Anion einer organischen oder anorganischen Säure ist und

(b) monomeren Carbonsäuren der allgemeinen Formel (III),

$$R^{10}\text{-CH=CR}^{11}\text{-COOH} \quad \text{(III)}$$

in denen $R^{10}$ und $R^{11}$ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen $R^{14}$, $R^{15}$ und $R^{16}$ Methylgruppen sind, Z eine NH-Gruppe und $A^{(-)}$ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.

Es ist erfindungsgemäß auch möglich, daß die verwendeten Zubereitungen mehrere, insbesondere zwei verschiedene Polymere gleicher Ladung und/oder jeweils ein ionisches und ein amphoteres Polymer enthalten.

Die Polymere sind in den erfindungsgemäß verwendeten Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Weiterhin können in den erfindungsgemäßen Zubereitungen Proteinhydrolysate, insbesondere kationisierte Proteinhydrolysate enthalten sein. Proteinhydrolysate sind Produktgemische, die durch sauer, basisch oder enzymatisch katalysierten Abbau von Proteinen (Eiweißen) erhalten werden.

Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seidenund Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex) und Crotein® (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon® (Cognis), Lexein® (Inolex), Crolastin® (Croda) oder Crotein® (Croda) vertrieben.

Kationisierte Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Ganz besonders bevorzugt sind die kationischen Proteinhydrolysate und -derivate auf pflanzlicher Basis.

Die Proteinhydrolysate oder deren Derivate sind in den erfindungsgemäß verwendeten Mitteln bevorzugt in Mengen von 0,1 bis 10 Gew.-%, bezogen auf das gesamte Mittel, enthalten. Mengen von 0,1 bis 5 Gew.-% sind besonders bevorzugt.

Die farberhaltende Wirkung kann erfindungsgemäß noch weiter durch eine 2-Pyrrolidinon-5-carbonsäure und deren Derivate gesteigert werden. Bevorzugt sind die Natrium-, Kalium-, Calcium-, Magnesium oder Ammoniumsalze, bei denen das Ammoniumion neben Wasserstoff eine bis drei $C_1$- bis $C_4$-Alkylgruppen trägt. Das Natriumsalz ist ganz besonders bevorzugt. Die eingesetzten Mengen in den erfindungsgemäßen Mitteln betragen 0,05 bis 10 Gew.%, bezogen auf das gesamte Mittel, besonders bevorzugt 0,1 bis 5, und insbesondere 0,1 bis 3 Gew.%.

Ebenfalls als vorteilhaft hat sich die Kombination des farberhaltenden Wirkstoffes mit kationischen Tensiden erwiesen.

Erfindungsgemäß bevorzugt sind dabei kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Ebenfalls als vorteilhaft hat sich die Kombination des farberhaltenden Wirkstoffes mit Vitaminen, Provitaminen und Vitaminvorstufen sowie deren Derivaten erwiesen.

Dabei sind erfindungsgemäß solche Vitamine, Pro-Vitamine und Vitaminvorstufen bevorzugt, die üblicherweise den Gruppen A, B, C, E, F und H zugeordnet werden.

Zur Gruppe der als **Vitamin A** bezeichneten Substanzen gehören das Retinol (Vitamin $A_1$) sowie das 3,4-Didehydroretinol (Vitamin $A_2$). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Zubereitungen enthalten die Vitamin A-Komponente bevorzugt in Mengen von 0,05-1 Gew.-%, bezogen auf die gesamte Zubereitung.

Zur **Vitamin B-Gruppe** oder zu dem Vitamin B-Komplex gehören u. a.

- Vitamin $B_1$ (Thiamin)

- Vitamin $B_2$ (Riboflavin)
- Vitamin $B_3$. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendetenen Mitteln bevorzugt in Mengen von 0,05 bis 1 Gew.-%, bezogen auf das gesamte Mittel, enthalten ist.
- Vitamin $B_5$ (Pantothensäure und Panthenol). Im Rahmen dieser Gruppe wird bevorzugt das Panthenol eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin $B_5$-Typs sind in den erfindungsgemäß verwendeten Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin $B_6$ (Pyridoxin sowie Pyridoxamin und Pyridoxal).

**Vitamin C** (Ascorbinsäure). Vitamin C wird in den erfindungsgemäß verwendeten Mitteln bevorzugt in Mengen von 0,1 bis 3 Gew.-%, bezogen auf das gesamte Mittel eingesetzt. Die Verwendung in Form des Palmitinsäureesters, der Glucodide oder Phosphate kann bevorzugt sein. Die Verwendung in Kombination mit Tocopherolen kann ebenfalls bevorzugt sein.

**Vitamin E** (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäß verwendeten Mitteln bevorzugt in Mengen von 0,05-1 Gew.-%, bezogen auf das gesamte Mittel, enthalten.

**Vitamin F.** Unter dem Begriff "Vitamin F" werden üblicherweise essentielle Fettsäuren, insbesondere Linolsäure, Linolensäure und Arachidonsäure, verstanden.

**Vitamin H.** Als Vitamin H wird die Verbindung (3a*S*,4*S*, 6a*R*)-2-Oxohexahydrothienol[3,4-*d*]-imidazol-4-valeriansäure bezeichnet, für die sich aber zwischenzeitlich der Trivialname Biotin durchgesetzt hat. Biotin ist in den erfindungsgemäß verwendeten Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäß verwendeten Mittel Vitamine, Provitamine und Vitaminvorstufen aus den Gruppen A, B, E und H.

Panthenol und seine Derivate sowie Nicotinsäureamid und Biotin sind besonders bevorzugt.

Schließlich läßt sich die Wirkung des farberhaltenden Wirkstoff/Wirkstoffgemisches auch durch den kombinierten Einsatz mit Pflanzenextrakten steigern.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders für die erfindungsgemäße Verwendung geeignet sind die Extrakte aus Grünem Tee, Mandel, Aloe Vera, Kokosnuß, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Der erfindungsgemäße farberhaltende Wirkstoff/Wirkstoffgemisch kann prinzipiell dem Färbemittel direkt zugegeben werden. Bevorzugt erfolgt das Aufbringen des farberhaltenden Wirkstoff/Wirkstoffgemisches auf die gefärbte keratinische Faser aber in einem getrennten Schritt entweder direkt im Anschluß an den eigentlichen Färbevorgang oder in getrennten Behandlungen, gegebenenfalls auch Tage oder Wochen nach dem Färbevorgang. Selbstverständlich umfaßt die erfindungsgemäße Lehre auch die Erkenntnis, daß eine wiederholte Anwendung des farberhaltenden Wirkstoffes/Wirkstoffgemisches, wenn diese wiederholt in größerem zeitlichen Abstand (Tage bis Wochen) vom eigentlichen Färbevorgang angewendet wird, eine Vorbehandlung vor dem eigentlichen Färbevorgang darstellt.

Der Begriff Färbevorgang umfaßt dabei alle dem Fachmann bekannten Verfahren, bei denen auf das, gegebenenfalls angefeuchtete, Haar ein Färbemittel aufgebracht wird und dieses entweder für eine Zeit zwischen wenigen Minuten und ca. 45 Minuten auf dem Haar belassen und anschließend mit Wasser oder einem tensidhaltigen Mittel ausgespült wird oder ganz auf dem Haar belassen wird. Es wird in diesem Zusammenhang ausdrücklich auf die bekannten Monographien, z. B. Kh. Schrader, Grundlagen und Rezepturen der Kosmetika, 2. Auflage, Hüthig Buch Verlag, Heidelberg, 1989, verwiesen, die das entsprechende Wissen des Fachmannes wiedergeben.

Hinsichtlich der Art, gemäß der der erfindungsgemäße farberhaltende Wirkstoff/Wirkstoffgemisch auf die keratinische Faser, insbesondere das menschliche Haar, aufgebracht wird, bestehen keine prinzipiellen Einschränkungen. Als Konfektionierung dieser Zubereitungen sind beispielsweise Cremes, Lotionen, Lösungen, Wässer, Emulsionen wie W/O-, O/W-, PIT-Emulsionen (Emulsionen nach der Lehre der Phaseninversion, PIT genannt), Mikroemulsionen und multiple Emulsionen, Gele, Sprays, Aerosole und Schaumaerosole geeignet. Diese werden in der Regel auf wäßriger oder wäßrig-alkoholischer Basis formuliert. Als alkoholische Komponente kommen dabei niedere Alkanole sowie Polyole wie Propylenglykol und Glycerin zum Einsatz. Ethanol und Isopropanol sind bevorzugte Alkohole. Wasser und Alkohol können in der wäßrig alkoholischen Basis in einem Gewichtsverhältnis von 1 : 10 bis 10 : 1 vorliegen. Wasser sowie wäßrig-alkoholische Mischungen, die bis zu 50 Gew.-%, insbesondere bis zu 25 Gew.-%, Alkohol, bezogen auf das Gemisch Alkohol/Wasser, enthalten, können erfindungsgemäß bevorzugte Grundlagen sein. Der pH-Wert dieser Zubereitungen kann prinzipiell bei Werten von 2 - 11 liegen. Er liegt bevorzugt zwischen 2 und 7, wobei Werte von 3 bis 5 besonders bevorzugt sind. Zur Einstellung dieses pH-Wertes kann praktisch jede für kosmetische Zwecke verwendbare Säure oder Base verwendet werden. Üblicherweise werden als Säuren Genußsäuren verwendet. Unter Genußsäuren werden solche Säuren verstanden, die im Rahmen der üblichen Nahrungsaufnahme aufgenommen werden und positive Auswirkungen auf den menschlichen Organismus haben. Genußsäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt. Bevorzugte Basen sind Ammoniak, Alkalihydroxide, Triethanolamin, N,N,N',N'-Tetrakis-(2-hydroxypropyl)-ethylendiamin sowie alkalisch reagierende Aminosäuren wie Ornithin, Histidin und bevorzugt Arginin.

Auf dem Haar verbleibende Zubereitungen haben sich als besonders wirksam erwiesen und können daher bevorzugte Ausführungsformen der erfindungsgemäßen Lehre darstellen. Unter auf dem Haar verbleibend werden erfindungsgemäß solche Zubereitungen verstanden, die nicht im Rahmen der Behandlung nach einem Zeitraum von wenigen Sekunden bis zu einer Stunde mit Hilfe von Wasser oder einer wäßrigen Lösung wieder aus dem Haar ausgespült werden. Vielmehr verbleiben die Zubereitungen bis zur nächsten Haarwäsche, d. h. in der Regel mehr als 12 Stunden, auf dem Haar.

Gemäß einer bevorzugten Ausführungsform werden diese Zubereitungen als Haarkur oder Haar-Conditioner formuliert. Die erfindungsgemäßen Zubereitungen gemäß dieser Ausführungsform können nach Ablauf dieser Einwirkzeit mit Wasser oder einem zumindest überwiegend wasserhaltigen Mittel ausgespült werden; bevorzugt werden sie jedoch, wie oben ausgeführt, auf dem Haar belassen. Dabei kann es bevorzugt sein, die erfindungsgemäße Zubereitung vor der Anwendung eines reinigenden Mittels, eines Wellmittels oder anderen Haarbehandlungsmitteln auf das Haar aufzubringen. In diesem Falle dient die erfindungsgemäße Zubereitung als Farbschutz für die nachfolgenden Anwendungen.

Gemäß weiteren Ausführungsformen kann es sich bei den erfindungsgemäßen Mitteln aber beispielsweise auch um reinigende Mittel wie Shampoos, pflegende Mittel wie Spülungen, festigende Mittel wie Haarfestiger, Schaumfestiger, Styling Gels und Fönwellen, dauerhafte Verformungsmittel wie Dauerwell- und Fixiermittel sowie insbesondere im Rahmen eines Dauerwellverfahrens oder Färbeverfahrens eingesetzte Vorbehandlungsmittel oder Nachspülungen handeln.

Neben dem erfindungsgemäß zwingend erforderlichen farberhaltenden Wirkstoff/Wirkstoffgemisch und den weiteren, oben genannten bevorzugten Komponenten können diese Zubereitungen prinzipiell alle weiteren, dem Fachmann für solche kosmetischen Mittel bekannten Komponenten enthalten.

Weitere Wirk-, Hilfs- und Zusatzstoffe sind beispielsweise

- nichtionogene Tenside wie beispielsweise Alkylphenolpolyglycolether, Fettsäurepolyglycolester, Fettsäureamidpolyglycolether, Fettaminpolyglycolether, alkoxylierte Triglyceride, wie insbesondere ethoxyliertes Rizinusöl, Alk(en)yloligoglucoside, Fettsäure-N-alkylglucamide, Polyolfettsäureester, Zuckerester, Sorbitanester und Polysorbate. Sofern die nichtionischen Tenside Polyglycoletherketten enthalten, können sie eine konventionelle oder eingeengte Homologenverteilung aufweisen.
- anionische Tenside, insbesondere Alkylsulfate, Alkylpolyglykolethersulfate und Ethercarbonsäuren mit 10 bis 18 C-Atomen in der Alkylgruppe und bis zu 12 Glykolethergruppen im Molekül, Seifen sowie Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- zwitterionische Tenside, insbesondere die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethyl-carboxymethylglycinat,
- ampholytische Tenside wie beispielsweise N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkyl-aminoessigsäuren mit jeweils etwa 8 bis 18 C-Atomen in der Alkylgruppe,
- nichtionische Polymere wie beispielsweise Vinylpyrrolidon/Vinylacrylat-Copoly mere, Polyvinylpyrrolidon und Vinylpyrrolidon/Vinylacetat-Copolymere und Polysiloxane,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline, sowie Silikonöle,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- symmetrische und unsymmetrische, lineare und verzweigte Dialkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether und Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tertbutylether, Di-iso-pentylether, Di-3-ethyldecylether, tert.-Butyl-n-octylether, iso-Pentyl-n-octylether und 2-Methyl-pentyl-n-octylether,
- Fettalkohole, insbesondere lineare und/oder gesättigte Fettalkohole mit 8 bis 30 C-Atomen, und Monoester der Fettsäuren mit Alkoholen mit 6 bis 24 C-Atomen,
- faserstrukturverbessernde Wirkstoffe, insbesondere Mono-, Di- und Oligosaccharide wie beispielsweise Glucose, Galactose, Fructose, Fruchtzucker und Lactose,
- konditionierende Wirkstoffe wie Paraffinöle, pflanzliche Öle, z. B. Sonnenblumenöl, Orangenöl, Mandelöl, Weizenkeimöl und Pfirsichkernöl sowie Phospholipide, beispielsweise Sojalecithin, Ei-Lecithin und Kephaline,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- weitere Substanzen zur Einstellung des pH-Wertes, wie beispielsweise $\alpha$- und $\beta$-Hydroxycarbonsäuren
- Wirkstoffe wie Allantoin und Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, $\beta$-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,

- Pigmente,
- Reduktionsmittel wie z. B. Thioglykolsäure und deren Derivate, Thiomilchsäure, Cysteamin, Thioäpfelsäure und $\alpha$-Mercaptoethansulfonsäure,
- Treibmittel wie Propan-Butan-Gemische, $N_2O$, Dimethylether, $CO_2$ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. die oben genannte Monographie von Kh. Schrader verwiesen.

Wie bereits oben erwähnt ist es im Rahmen der erfindungsgemäßen Lehre auch möglich, wenngleich weniger bevorzugt, den farberhaltenden Wirkstoff/Wirkstoffgemisch direkt in die Färbe- oder Tönungsmittel einzuarbeiten.

Die Zusammensetzung des Färbe- oder Tönungsmittels unterliegt keinen prinzipiellen Einschränkungen. Als Farbstoff(vorprodukt)e können

- Oxidationsfarbstoffvorprodukte vom Entwickler- und Kuppler-Typ,
- natürliche und synthetische direktziehende Farbstoffe und
- Vorstufen naturanaloger Farbstoffe, wie Indol- und Indolin-Derivate, sowie Mischungen von Vertretern einer oder mehrerer dieser Gruppen eingesetzt werden.

Als Oxidationsfarbstoffvorprodukte vom Entwickler-Typ werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt. Geeignete Entwicklerkomponenten sind beispielsweise p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 741 bzw. WO 94/08970 wie z. B. 4,5-Diamino-1-(2'-hydroxyethyl)-pyrazol. Besonders vorteilhafte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin.

Als Oxidationsfarbstoffvorprodukte vom Kuppler-Typ werden in der Regel m-Phenylendiaminderivate, Naphthole, Resorcin und Resorcinderivate, Pyrazolone und m-Aminophenolderivate verwendet. Beispiele für solche Kupplerkomponenten sind

- m-Aminophenol und dessen Derivate wie beispielsweise 5-Amino-2-methylphenol, 5-(3-Hydroxypropylamino)-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,

- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol,

Besonders geeignete Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Direktziehende Farbstoffe sind üblicherweise Nitrophenylendiamine, Nitroaminophenole, Azofarbstoffe, Anthrachinone oder Indophenole. Besonders geeignete direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsδure, 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol.

In der Natur vorkommende direktziehende Farbstoffe sind beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Als Vorstufen naturanaloger Farbstoffe werden beispielsweise Indole und Indoline sowie deren physiologisch verträgliche Salze verwendet. Bevorzugt werden solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. Besonders vorteilhafte Eigenschaften haben 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin sowie 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin sowie N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden.

Bei der Verwendung von Farbstoff-Vorstufen vom Indolin- oder Indol-Typ kann es bevorzugt sein, diese zusammen mit mindestens einer Aminosäure und/oder mindestens einem Oligopeptid einzusetzen. Bevorzugte Aminosäuren sind Aminocarbonsäuren, insbesondere α-Aminocarbonsäuren und ω-Aminocarbonsäuren. Unter den α-Aminocarbonsäuren sind wiederum Arginin, Lysin, Ornithin und Histidin besonders bevorzugt. Eine ganz besonders bevorzugte Aminosäure ist Arginin, insbesondere in freier Form, aber auch als Hydrochlorid eingesetzt.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt. Zu diesem Zweck enthalten die Färbemittel Alkalisierungsmittel, üblicherweise Alkali - oder Erdalkalihydroxide, Ammoniak oder organische Amine. Bevorzugte Alkalisierungsmittel sind Monoethanolamin, Monoisopropanolamin, 2-Amino-2-methylpropanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Erfolgt die Ausbildung der eigentlichen Haarfarben im Rahmen eines oxidativen Prozesses, so können übliche Oxidationsmittel, wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel. So können die Enzyme (Enzymklasse 1: Oxidoreduktasen) Elektronen aus geeigneten Entwicklerkomponenten (Reduktionsmittel) auf Luftsauerstoff übertragen. Bevorzugt sind dabei Oxidasen wie Tyrosinase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Zweckmäßigerweise wird die Zubereitung des Oxidationsmittels dann unmittelbar vor dem Färben der Haare mit der Zubereitung mit den Farbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45, insbesondere 15 bis 30, Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann- gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Weiterhin kann die Ausbildung der Färbung dadurch unterstützt und gesteigert werden, daß dem Mittel bestimmte Metallionen zugesetzt werden. Solche Metallionen sind beispielsweise $Zn^{2+}$, $Cu^{2+}$, $Fe^{2+}$, $Fe^{3+}$, $Mn^{2+}$, $Mn^{4+}$, $Li^{+}$, $Mg^{2+}$, $Ca^{2+}$ und $Al^{3+}$. Besonders geeignet sind dabei $Zn^{2+}$, $Cu^{2+}$ und $Mn^{2+}$. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflußt werden.

Ein zweiter Gegenstand der Erfindung sind Mittel zur Behandlung von Fasern, insbesondere keratinischer Fasern, die eine Kombination aus

- einem der Wirkstoffe oder der Wirkstoffkombination (A) und
- einem Kohlenhydrat (B) enthalten.

Noch ein Gegenstand der Erfindung ist ein Verfahren zur Färbung von Fasern, insbesondere keratinischer, Fasern, bei dem in einem ersten Schritt die Fasern in üblicher Weise gefärbt werden und in einem zweiten Schritt ein Mittel auf die Fasern aufgetragen wird, das einen Wirkstoff oder Wirkstoff/Wirkstoffgemisch (A) enthält, wobei das Mittel gewünschtenfalls nach einer Einwirkzeit von 1 bis 5 Minuten wieder ausgespült wird.

Ein weiterer Gegenstand der Erfindung ist schließlich ein Verfahren zur Färbung von Fasern, insbesondere keratinischer Fasern, bei dem in einem ersten Schritt ein Mittel auf die Fasern aufgetragen wird, das einen Wirkstoff/Wirkstoffgemisch zur Erhöhung der Waschechtheit der Färbung enthält, und anschließend in einem zweiten Schritt die Fasern in üblicher Weise gefärbt werden. Im Rahmen dieses Verfahrens kann es bevorzugt sein, das erste Mittel in Form eines Sprays zu applizieren.

Anhand der nachfolgenden Beispiele wird der Gegenstand der Erfindung näher erläutert.

**Wirkungsnachweis**

**Beispiele 1 - 4**

Alle Mengenangaben sind, soweit nicht anders angegeben, Gewichtsteile.
Beispiele 1 ist das Vergleichsbeispiel
Die Beispiele 2 - 4 betreffen erfindungsgemäße Zusammensetzungen.

**1. Haarspülung ohne Wirkstoff**

| | |
|---|---|
| Paraffinöl | 3,0 |
| Lanette® O[1] | 3,0 |
| Foryl® 100[2] | 0,2 |
| Cutina® CP[3] | 0,8 |
| Bienenwachs | 0,05 |
| PHB[4]-Methylester | 0,2 |
| PHB[4]-Propylester | 0,1 |
| Phenoxyethanol | 0,6 |
| Dehyquart®[5] A-CA | 3,0 |
| Glucose | 5,0 |
| Herbasol Destillat Kamille | 0,6 |
| Culminal®[6] MHPC 3000 (3% Quell) | 20.0 |
| Parfum | 0,15 |
| Wasser | ad 100 |
| pH-Wert ca. | 3,5 |

[1] Cetylstearylalkohol (INCI - Bezeichnung: Cetearyl Alcohol) (COGNIS)
[2] C12-C18 Fettalkohol mit 9 EO (INCI - Bezeichnung Laureth-10) (COGNIS)
[3] Cetylpalmitat (INCI:Bezeichnung: Cetyl Palmitate) (COGNIS)
[4] 4-Hydroxybenzoesäuremethyl, bzw. - propylester
[5] Cetyltrimethylammoniumchlorid (INCI - Bezeichnung: Cetrimoniumchloride) (COGNIS)
[6] Methylhydroxypropylcellulose (INCI - Bezeichnung: Hydroxypropylmethylcellulose) (Aqualon)

**Beispiel 2.**

| Haarspülung mit Wirkstoff | |
|---|---|
| Flexan® 130[7] | 1,0 |
| Paraffinöl | 3,0 |
| Lanette® O | 3,0 |
| Foryl® 100 | 0,2 |
| Cutina® CP | 0,8 |
| Bienenwachs | 0,05 |
| PHB-Methylester | 0,2 |
| PHB-Propylester | 0,1 |
| Phenoxyethanol | 0,6 |
| Dehyquart® A-CA | 3,0 |
| Glucose | 5,0 |
| Herbasol Destillat Kamille | 0,6 |
| Culminal® MHPC 3000 (3% Quell) | 20.0 |

[7] Polystyrolsulfonat (INCI - Bezeichnung: Sodium Polystyrene Sulfonate) (National Starch)

(fortgesetzt)

| Haarspülung mit Wirkstoff | |
|---|---|
| Parfüm | 0,15 |
| Wasser | ad 100 |
| pH-Wert ca. | 3,5 |

**Beispiel 3**

| Haarspülung mit Wirkstoff | |
|---|---|
| Surfadone ®[8]LP 300 | 1,0 |
| Paraffinöl | 3,0 |
| Lanette® O | 3,0 |
| Foryl® 100 | 0,2 |
| Cutina® CP | 0,8 |
| Bienenwachs | 0,05 |
| PHB-Methylester | 0,2 |
| PHB-Propylester | 0,1 |
| Phenoxyethanol | 0,6 |
| Dehyquart® A-CA | 3,0 |
| Glucose | 5,0 |
| Herbasol Destillat Kamille | 0,6 |
| Culminal® MHPC 3000 (3% Quell) | 20.0 |
| Parfüm | 0,15 |
| Wasser | ad 100 |
| pH-Wert ca. | 3,5 |

[8] N-Dodecylpyrrolidon-2 (INCI - Bezeichnung: Lauryl Pyrrolidone) (ISP)

**Beispiel 4**

| Haarspülung mit Wirkstoff | |
|---|---|
| Dow Corning® 949[9] | 0,5 |
| Paraffinöl | 3,0 |
| Lanette® O | 3,0 |
| Foryl® 100 | 0,2 |
| Cutina® CP | 0,8 |
| Bienenwachs | 0,05 |
| PHB-Methylester | 0,2 |
| PHB-Propylester | 0,1 |
| Phenoxyethanol | 0,6 |
| Dehyquart® A-CA | 3,0 |
| Glucose | 5,0 |
| Herbasol Destillat Kamille | 0,6 |
| Culminal® MHPC 3000 (3% Quell) | 20.0 |
| parfum | 0,30 |
| Wasser | ad 100 |

[9] kationische Emulsion enthaltend Amodimethicon, Cetyltrimethylammoniumchlorid und Trideceth-12 (INCI - Bezeichnung: Amodimethicone, Cetrimonium Chloride AND Trideceth-12) (Dow Corning Corporation)

(fortgesetzt)

| Haarspülung mit Wirkstoff | |
|---|---|
| pH-Wert ca. | 3,5 |

In der nachstehenden Tabelle 1 sind die Ergebnisse bei der Nachbehandlung einer Coloration im Hinblick auf die Wasch- und Farbstabilität bei Verwendung der erfindungsgemäßen wirkstoffhaltigen Mittel im Vergleich zu einem Wirkstoff freien Mittel (Beispiel 1) wiedergegeben. Hierzu wurden Haarsträhnen mit Poly Country Colors Herbstrot Nr. 45 und Granatrot Nr. 58 coloriert, mit den Mittel der Beispiele 1-4 behandelt und anschließend vor den Haarwäschen farbmetrisch vermessen und nach den Haarwäschen wieder vermessen. Es wurden insgesamt jeweils 6 Haarwäschen im Ultraschalbad durchgeführt. Als Vergleich diente jeweils eine mit dem wirkstofffreien Mittel (Beispiel 1) behandelte Haarsträhne.

Tabelle 1

| Waschstabilität und Farbstabilität der Coloration | | | | |
|---|---|---|---|---|
| Poly Country Colors | Herbstrot Nr. 45 | | Granatrot Nr. 58 | |
| Zusammensetzung der Haarspülung | Ungeschädigte Strähne | Leicht geschädigte Strähne | UngeSchädigte Strähne | Leicht geschädigte Strähne |
| | % Farbe | % Farbe | % Farbe | % Farbe |
| Beispiel 1 | 61,43 | 64,43 | 84,57 | 71,78 |
| Beispiel 2 | 62,00 | 69,68 | 88,81 | 90,36 |
| Beispiel 3 | 64,78 | 72,22 | 87,49 | 89,14 |
| Beispiel 4 | 63,57 | 70,71 | 89,05 | 82,70 |

Die Ergebnisse der Tabelle I zeigen, dass die Wasch- und Farbstabilität durch die erfindungsgemäße Verwendung der wirkstoffhaltigen Mittel deutlich gesteigert werden kann.

**Weitere Anwendungsbeispiele**

1. Haarspülung

| | |
|---|---|
| Eumulgin® B2[1] | 0,3 |
| Cetyl/Stearylalkohol | 3,3 |
| Isopropylmyristat | 0,5 |
| Lamesoft® PO 65[4] | 0,5 |
| Dehyquart®A-CA[2] | 2,0 |
| Salcare®SC 96[5] | 1,0 |
| Citronensäure | 0,4 |
| Gluadin® WQ[6] | 2,0 |
| Flexan® 130 | 2,0 |
| Pyridoxin | 1,0 |
| D-Glucose | 0,8 |
| D-Glucose-6-phosphat | 0,2 |

1. Cetylstearylalkohol + 20 EO (INCI-Bezeichnung: Ceteareth-20) (COGNIS)
2. Trimethylhexadecylammoniumchlorid ca. 25% Aktivsubstanz (INCI-Bezeichnung: Cetrimonium Chloride) (COGNIS)
4. Gemisch aus Alkylpolyglycosid und Fettsäuremonoglycerid (INCI-Bezeichnung: Coco-Glucoside (and) Glyceryl Oleate)
5. N,N,N-Trimethyl-2[(methyl-1-oxo-2-propenyl)oxy]-Ethanaminiumchlorid-Homo-polymer (50 % Aktivsubstanz; INCI-Bezeichnung: Polyquaternium-37 (and) Pro-pylenglycol Dicaprilate Dicaprate (and) PPG-1 Trideceth-6) (ALLIED COLLOIDS)
6. Kationisiertes Weizenproteinhydrolysat ca. 31% Aktivsubstanz (INCI-Bezeichnung: Laurdimonium Hydroxypropyl Hydrolyzed Wheat Protein) (COGNIS)

(fortgesetzt)

| | |
|---|---|
| Weinsäure | 0,7 |
| Phenonip®[3] | 0,8 |
| Wasser | ad 100 |

[3] Hydroxybenzoesäuremethylester-Hydroxybenzoesäureethylester-Hydroxybenzoesäurepropylester-Hydroxybenzoesäurebutylester-Phenoxyethanol-Gemisch (ca. 28 % Aktivsubstanz; INCI-Bezeichnung: Phenoxyethanol, Methylparaben, Ethylparaben, Propylparaben, Butylparaben) (NIPA)

2. Haarspülung

| | |
|---|---|
| Eumulgin® B2 | 0,3 |
| Cetyl/Stearylalkohol | 3,3 |
| Isopropylmyristat | 0,5 |
| Paraffinöl perliquidum 15 cSt. DAB 9 | 0,3 |
| Dehyquart®L 80[7] | 0,4 |
| Lamesoft® PO 65 | 1,5 |
| Cosmedia Guar® C 261[8] | 1,5 |
| Dow Corning 1401 | 1,0 |
| Surfadone LP-100 | 2,0 |
| Promois® Milk-CAQ[9] | 3,0 |
| Citronensäure | 0,4 |
| D-Erythrose | 0,5 |
| Phenonip® | 0,8 |
| Wasser | ad 100 |

[7] Bis(cocoylethyl)-hydroxyethyl-methyl-ammonium-methosulfat (ca. 76 % Aktivsubstanz in Propylenglykol; INCI-Bezeichnung: Dicocoylethyl Hydroxyethylmonium Methosulfat, Propylene Glycol) (COGNIS)

[8] Guarhydroxypropyltrimethylammonium Chlorid; INCI-Bezeichnung: Guar Hydroxy-propyl Trimonium Chloride (COGNIS)

[9] INCI-Bezeichnung: Cocodimonium Hydroxypropyl Hydrolyzed Casein (SEIWA KASEI)

3. Haarkur

| | |
|---|---|
| Dehyquart® F75[10] | 4,0 |
| Cetyl/Stearylalkohol | 4,0 |
| Paraffinöl perliquidum 15 cSt DAB 9 | 1,5 |
| Dehyquart®A-CA | 4,0 |
| Lamesoft® PO 65 | 1,0 |
| Salcare®SC 96 | 1,5 |
| Glucosaminhydrochlorid | 2,5 |
| Glyoxylsäure | 0,5 |
| Amisafe-LMA-60®[11] | 1,0 |
| Gluadin®W 20[12] | 3,0 |
| Surfadone LP-300 | 1,0 |
| Germall® 115[13] | 1,0 |
| Citronensäure | 0,15 |

[10] Fettalkohole-Methyltriethanolammoniummethylsulfatdialkylester-Gemisch (INCI-Bezeichnung: Distearoylethyl Hydroxyethylmonium Methosulfate, Cetearyl Alcohol) (COGNIS)

[11] INCI-Bezeichnung Hydroxypropyl Arginine Lauryl/Myristyl Ether HCl (Ajinomoto)

[12] Weizenproteinhydrolysat (20 % Aktivsubstanz in Wasser; INCI-Bezeichnung: Aqua (and) Hydrolized Wheat Protein (and) Sodium Benzoate (and) Phenoxyethanol (and) Methylparaben (and) Propylparaben) (COGNIS)

[13] INCI-Bezeichnung: Imidazolidinyl Urea (Sutton Laboratories)

(fortgesetzt)

| | |
|---|---|
| Phenonip® | 0,8 |
| Wasser | ad 100 |

4. Haarkur

| | |
|---|---|
| Dehyquart® L80 | 2,0 |
| Cetyl/Stearylalkohol | 6,0 |
| Paraffinöl perliquidum 15 cSt DAB 9 | 2,0 |
| Rewoquat®W 75[14] | 2,0 |
| Cosmedia Guar® C261 | 0,5 |
| Lamesoft® PO 65 | 0,5 |
| Sepigel®305[15] | 3,5 |
| Honeyquat® 50[16] | 1,0 |
| Gluadin® WQ | 2,5 |
| Gluadin®W 20 | 3,0 |
| Rhamnose | 1,0 |
| Dow Corning 949 | 2,5 |
| Citronensäure | 0,15 |
| Phenonip® | 0,8 |
| Wasser | ad 100 |

14. 1-Methyl-2-nortalgalkyl-3-talgfettsäureamidoethylimidazolinium-methosulfat (ca. 75 % Aktivsubstanz in Propylenglykol; INCI-Bezeichnung: Quaternium-27, Propylene Glycol) (WITCO)

15. Copolymer aus Acrylamid und 2-Acrylamido-2-methylpropansulfonsäure (INCI-Bezeichnung: Polyacrylamide (and) $C_{13}$-$C_{14}$ Isoparaffin (and) Laureth-7) (SEPPIC)

16. INCI - Bezeichnung: Hydroxypropyltrimonium Honey (BROOKS)

5. Haarkur

| | |
|---|---|
| Dehyquart® F75 | 0,3 |
| Salcare®SC 96 | 5,0 |
| Gluadin® WQ | 1,5 |
| Lamesoft® PO 65 | 0,5 |
| Dow Corning®200 Fluid, 5 cSt.[17] | 1,5 |
| Gafquat®755N[18] | 1,5 |
| Sorbit | 1,5 |
| Flouwet EA 093 | 2,5 |
| Biodocarb® [19] | 0,02 |
| Parfümöl | 0,25 |
| Wasser | ad 100 |

17. Polydimethylsiloxan (INCI-Bezeichnung: Dimethicone) (DOW CORNING)

18. Dimethylaminoethylmethacrylat-Vinylpyrrolidon-Copolymer, mit Diethylsulfat quaterniert (19 % Aktivsubstanz in Wasser; INCI-Bezeichnung: Polyquaternium-11) (GAF)

19. 3-Iod-2-propinyl-n-butylcarbamat (INCI-Bezeichnung: Iodopropynyl Butylcarbamate) (MILKER & GRÜNING)

6. Haarkur

| | |
|---|---|
| Sepigel®305 | 5,0 |

(fortgesetzt)

| | |
|---|---|
| Dow Corning®Q2-5220[20] | 1,5 |
| Promois® Milk Q[21] | 3,0 |
| Lamesoft® PO 65 | 0,5 |
| Polymer P 1 entsprechend DE 3929173 | 0,6 |
| Genamin®DSAC[22] | 0,3 |
| L-Arabitol | 1,8 |
| Dow Corning X2-8194 | 2,0 |
| Surfadone LP-300 | 0,2 |
| Phenonip® | 0,8 |
| Parfümöl | 0,25 |
| Wasser | ad 100 |

20. Silicon-Glykol-Copolymer (INCI-Bezeichnung: Dimethicone Copolyol) (DOW CORNING)
21. INCI-Bezeichnung Hydroxypropyltrimonium Hydrolyzed Casein ca. 30% Aktivsubstanz (SEIWA KASEI)
22. Dimethyldistearylammoniumchlorid (INCI-Bezeichnung: Distearyldimonium Chloride) (CLARIANT)

7. Shampoo

| | |
|---|---|
| Texapon® NSO[23] | 40,0 |
| Dehyton® G[24] | 6,0 |
| Polymer JR 400®[25] | 0,5 |
| Cetiol® HE[26] | 0,5 |
| Ajidew® NL 50[27] | 1,0 |
| Lamesoft® PO 65 | 3,0 |
| Gluadin® WQT[28] | 2,5 |
| Gluadin® W 20 | 0,5 |
| Dow Corning 929 | 0,5 |
| Surfadone LP-300 | 0,5 |
| Panthenol (50%) | 0,3 |
| Pentaerythrit | 2,0 |
| Vitamin E | 0,1 |
| Vitamin H | 0,1 |
| Glutaminsäure | 0,2 |
| Citronensäure | 0,5 |
| Natriumbenzoat | 0,5 |
| Parfüm | 0,4 |
| NaCl | 0,5 |
| Wasser | ad 100 |

23. Natriumlaurylethersulfat ca. 28% Aktivsubstanz (INCI - Bezeichnung: Sodium Laureth Sulfate) (COGNIS)
24. INCI - Bezeichnung: Sodium Cocoamphoacetate, ca. 30% Aktivsubstanz in Wasser) (COGNIS)
25. quaternierte Hydroxyethylcellulose (INCI - Bezeichnung: Polyquaternium-10) (UNION CARBIDE)
26. Polyol-Fettsäure-Ester (INCI - Bezeichnung: PEG-7 Glyceryl Cocoate) (COGNIS)
27. Natrium-Salz der 2-Pyrrolidinon-5-carbonsäure (50% Aktivsubstanz: INCI-Bezeichnung: Sodium PCA) (AJINOMOTO)
28. INCI-Bezeichnung: Hydroxypropyltrimonium Hydrolyzed Wheat Protein (COGNIS)

8. Shampoo

| | |
|---|---|
| Texapon® NSO | 43,0 |

(fortgesetzt)

| | |
|---|---|
| Dehyton® K[29] | 10,0 |
| Plantacare® 1200 UP[30] | 4,0 |
| Lamesoft® PO 65 | 2,5 |
| Euperlan®PK 3000[31] | 1,6 |
| Arquad®316[32] | 0,8 |
| Polymer JR® 400 | 0,3 |
| Gluadin® WQ | 4,0 |
| Milchsäure | 0,5 |
| Glucose | 0,5 |
| Dow Corning 949 | 0,5 |
| Surfadone LP-300 | 2,5 |
| Glucamate®DOE 120[33] | 0,5 |
| Natriumchlorid | 0,2 |
| Wasser | ad 100 |

29. INCI - Bezeichnung: Cocamidopropyl Betaine ca. 30% Aktivsubstanz (COGNIS)

30. C 12 - C 16 Fettalkoholglycosid ca. 50% Aktivsubstanz (INCI - Bezeichnung: Lauryl Glucoside) (COGNIS)

31. Flüssige Dispersion von perlglanzgebenden Substanzen und Amphotensid (ca. 62 % Aktivsubstanz; CTFA-Bezeichnung: Glycol Distearate (and) Glycerin (and) Laureth-4 (and) Cocoamidopropyl Betaine) (COGNIS)

32. Tri-$C_{16}$-alkylmethylammoniumchlorid (AKZO)

33. ethoxyliertes Methylglucosid-dioleat (CTFA-Bezeichnung: PEG-120 Methyl Glucose Dioleate) (AMERCHOL)

9. Shampoo

| | |
|---|---|
| Texapon®N 70[34] | 21,0 |
| Plantacare® 1200 UP | 8,0 |
| Lamesoft® PO 65 | 3,0 |
| Gluadin® WQ | 1,5 |
| Cutina® EGMS[35] | 0,6 |
| Honeyquat® 50 | 2,0 |
| Ajidew® NL 50 | 2,8 |
| Antil® 141[36] | 1,3 |
| Fructose | 0,5 |
| Dow Corning Q2-8177 | 2,0 |
| Flexan 130 | 0,5 |
| Natriumchlorid | 0,2 |
| Magnesiumhydroxid | ad pH 4,5 |
| Wasser | ad 100 |

34. Natriumlaurylethersulfat mit 2 Mol EO ca. 70% Aktivsubstanz (INCI - Bezeichnung: Sodium Laureth Sulfate) (COGNIS)

35. Ethylenglykolmonostearat (ca. 25-35% Monoester, 60-70% Diester; INCI:Bezeichnung: Glycol Stearate) (COGNIS)

36. Polyoxyethylen-propylenglykoldioleat (40 % Aktivsubstanz; INCI - Bezeichnung: Propylene Glycol (and) PEG-55 Propylene Glycol Oleate) (GOLDSCHMIDT)

10. Shampoo

| | |
|---|---|
| Texapon® K 14 S[37] | 50,0 |
| Dehyton® K | 10,0 |

37. Natriumlaurylmyristylethersulfat ca 28% Aktivsubstanz (INCI - Bezeichnung: Sodium Myreth Sulfate) (COGNIS)

(fortgesetzt)

| | |
|---|---|
| Plantacare® 818 UP[38] | 4,5 |
| Lamesoft® PO 65 | 2,0 |
| Polymer P1, entsprechend DE 39 29 973 | 0,6 |
| Cutina® AGS[39] | 2,0 |
| D-Panthenol | 0,5 |
| Fructose | 1,0 |
| Surfadone LP-100 | 1,0 |
| Salicylsäure | 0,4 |
| Natriumchlorid | 0,5 |
| Gluadin® WQ | 2,0 |
| Wasser | ad 100 |

38. C 8 - C 16 Fettalkoholglycosid ca. 50% Aktivsubstanz (INCI - Bezeichnung: Coco Glucoside) (COGNIS)
39. Ethylenglykolstearat (ca. 5-15% Monoester, 85-95% Diester; INCI - Bezeichnung: Glycol Distearate) (COGNIS)

11. Haarkur

| | |
|---|---|
| Celquat® L 200[40] | 0,6 |
| Luviskol® K30[41] | 0,2 |
| D-Panthenol | 0,5 |
| Polymer P1, entsprechend DE 39 29 973 | 0,6 |
| Dehyquart® A-CA | 1,0 |
| Lamesoft® PO 65 | 0,5 |
| Surfadone LP-300 | 1,0 |
| Isomalt | 1,0 |
| Asparaginsäure | 0,3 |
| Gluadin® W 40[42] | 1,0 |
| Natrosol® 250 HR[43] | 1,1 |
| Gluadin® WQ | 2,0 |
| Wasser | ad 100 |

40. quaterniertes Cellulose-Derivat (95 % Aktivsubstanz; CTFA-Bezeichnung: Polyquaternium-4) (DELFT NATIONAL)
41. Polyvinylpyrrolidon (95 % Aktivsubstanz; CTFA-Bezeichnung: PVP) (BASF)
42. Partialhydrolysat aus Weizen ca. 40% Aktivsubstanz (INCI - Bezeichnung: Hydrolyzed Wheat Gluten Hydrolyzed Wheat Protein) (COGNIS)
43. Hydroxyethylcellulose (AQUALON)

12. Färbecreme

| | |
|---|---|
| $C_{12-18}$-Fettalkohol | 1,2 |
| Lanette® O[44] | 4,0 |
| Eumulgin® B 2 | 0,8 |
| Cutina® KD 16[45] | 2,0 |
| Lamesoft® PO 65 | 4,0 |
| Natriumsulfit | 0,5 |
| L(+)-Ascorbinsäure | 0,5 |
| Ammoniumsulfat | 0,5 |
| 1,2-Propylenglykol | 1,2 |

44. Cetylstearylalkohol (INCI - Bezeichnung: Cetearyl Alcohol) (COGNIS)
45. Selbstemulgierendes Gemisch aus Mono- / Diglyceriden höherer gesättigter Fettsäuren mit Kaliumstearat (INCI - Bezeichnung: Glyceryl Stearate SE) (COGNIS)

(fortgesetzt)

| | |
|---|---|
| Polymer JR®400 | 0,3 |
| p-Aminophenol | 0,35 |
| p-Toluylendiamin | 0,85 |
| 2-Methylresorcin | 0,14 |
| 6-Methyl-m-aminophenol | 0,42 |
| Cetiol® OE[46] | 0,5 |
| Honeyquat® 50 | 1,0 |
| Ajidew® NL 50 | 1,2 |
| Gluadin® WQ | 1,0 |
| Dow Corning 949 | 1,0 |
| Flexan 130 | 0,7 |
| Arabit | 0,5 |
| Ammoniak | 1,5 |
| Wasser | ad 100 |

46. Di-n-octylether (INCI - Bezeichnung: Dicaprylyl Ether) (COGNIS)

13. Entwicklerdispersion für Färbecreme 12.

| | |
|---|---|
| Texapon® NSO | 2,1 |
| Wasserstoffperoxid (50%ig) | 12,0 |
| Turpinal® SL[47] | 1,7 |
| Latekoll® D[48] | 12,0 |
| Lamesoft® PO 65 | 2,0 |
| Gluadin® WQ | 0,3 |
| Salcare® SC 96 | 1,0 |
| Surfadone LP-300 | 2,0 |
| Asparaginsäure | 0,1 |
| Mannit | 0,8 |
| Wasser | ad 100 |

47. 1-Hydroxyethan-1,1-diphosphonsäure (60 % Aktivsubstanz; INCI - Bezeichnung: Etidronic Acid) (COGNIS)
48. Acrylester-Methacrylsäure-Copolymer (25 % Aktivsubstanz) (BASF)

14. Tönungsshampoo

| | |
|---|---|
| Texapon® N 70 | 14,0 |
| Dehyton® K | 10,0 |
| Akypo® RLM 45 NV[49] | 14,7 |
| Plantacare® 1200 UP | 4,0 |
| Lamesoft® PO 65 | 3,0 |
| Polymer P1, entsprechend DE 39 29 973 | 0,3 |
| Cremophor® RH 40[50] | 0,8 |
| Dow Corning 929 | 0,5 |
| Cellobiose | 0,3 |
| Saccharose | 0,3 |
| Elaidinsäure | 0,3 |

49. Laurylalkohol+4,5 Ethylenoxid-essigsäure-Natriumsalz (20,4 % Aktivsubstanz) (CHEM-Y)
50. Rizinus-Öl, hydriert + 45 Ethylenoxid (INCI - Bezeichnung: PEG-40 Hydrogenated Castor Oil) (BASF)

(fortgesetzt)

| | |
|---|---|
| Farbstoff C.I. 12 719 | 0,02 |
| Farbstoff C.I. 12 251 | 0,02 |
| Farbstoff C.I. 12 250 | 0,04 |
| Farbstoff C.I. 56 059 | 0,03 |
| Konservierung | 0,25 |
| Parfümöl | q.s. |
| Eutanol® G[51] | 0,3 |
| Gluadin® WQ | 1,0 |
| Honeyquat® 50 | 1,0 |
| Salcare® SC 96 | 0,5 |
| Wasser | ad 100 |

51. 2-Octyldodecanol (Guerbet-Alkohol) (INCI - Bezeichnung: Octyldodecanol) (COGNIS)

15. Cremedauerwelle

| Wellcreme | |
|---|---|
| Plantacare® 810 UP[52] | 5,0 |
| Thioglykolsäure | 8,0 |
| Turpinal® SL | 0,5 |
| Ammoniak (25%ig) | 7,3 |
| Ammoniumcarbonat | 3,0 |
| Cetyl/Stearyl-Alkohol | 5,0 |
| Lamesoft® PO 65 | 0,5 |
| Guerbet-Alkohol | 4,0 |
| Dow Corning 929 | 0,3 |
| Salcare® SC 96 | 3,0 |
| Gluadin® WQ | 2,0 |
| Lactose | 0,2 |
| Parfümöl | q.s. |
| Wasser | ad 100 |

52. $C_8$-$C_{10}$-Alkylglucosid mit Oligomerisationsgrad 1,6 (ca. 60% Aktivsubstanz) (COGNIS)

| Fixierlösung | |
|---|---|
| Plantacare® 810 UP | 5,0 |
| gehärtetes Rizinusöl | 2,0 |
| Lamesoft® PO 65 | 1,0 |
| Kaliumbromat | 3,5 |
| Nitrilotriessigsäure | 0,3 |
| Zitronensäure | 0,2 |
| Merquat® 550[53] | 0,5 |
| Dow Corning 929 | 0,5 |
| Surfadone LP-100 | 2,0 |
| Hydagen® HCMF[54] | 0,5 |
| Weinsäure | 0,5 |
| Gluadin® WQ | 0,5 |

53. Dimethyldiallylammoniumchlorid-Acrylamid-Copolymer (8 % Aktivsubstanz; INCI - Bezeichnung: Polyquarternium 7) (MOBIL OIL)
54. Chitosan Pulver (INCI - Bezeichnung: Chitosan) (COGNIS)

(fortgesetzt)

| Fixierlösung | |
|---|---|
| Maltose | 0,3 |
| Parfümöl | q.s. |
| Wasser | ad 100 |

16. Weitere Ausführungsbeispiele

Die Mengenangaben verstehen sich, soweit nichts anderes vermerkt ist, in Gewichtsprozent.

16.1 Färbung

*16.1.1 Herstellung der Gelgrundlage*

Es wurden die folgenden Gelgrundlagen hergestellt:

| Rohstoff | VG (Vergleichsrezeptur) | EG (Erfindungsgemäße Rezeptur) |
|---|---|---|
| Edenor® PK1805[55] | 6,8 | 6,8 |
| Texapon® NSO | 3,5 | 3,5 |
| 1,2-Propylenglykol | 6,8 | 6,8 |
| Kokoslorol[56] | 6,8 | 6,8 |
| Dehydol® LS2[57] | 12,0 | 12,0 |
| Isopropanol | 14,5 | 14,5 |
| Natriumsulfit | 0,2 | 0,2 |
| Turpinal® SL | 0,2 | 0,2 |
| Monoethanolamin | 5,5 | 5,5 |
| Farbstoff(vorprodukt)e (Zusammensetzung gemäß Punkt 16.1.2) | 20mMol | 20mMol |
| Dow Corning® 949 | -- | 0,5 |
| Glucose | -- | 0,5 |
| Surfadone® LP-300 | -- | 1,0 |
| Wasser | ad 100 | ad 100 |

55. Ölsäure (INCI - Bezeichnung: Olic Acid) (Cognis)
56. $C_{12-18}$-Fettalkohol (INCI-Bezeichnung: Coconut Alcohol) (Cognis)
57. $C_{12-14}$-Fettalkohol mit ca. 2-EO-Einheiten (INCI-Bezeichnung: Laureth-2) (Cognis)

*16.1.2 Farbstoffzubereitungen*

Es wurden die folgenden Farbstoff(vorprodukt)e in den in der Tabelle angegebenen Molverhältnissen eingesetzt:

E1 : 4,5-Diamino-1-(2-hydroxyethylpyrazol)
K1 : 5-Amino-2-methylphenol
K2 : 1-Methyl-2-hydroxy-4-(2-hydroxyethyl)aminobenzol
W1 : 4-Formyl-1-methylchinolinium-p-toluolsulfonat
W2 : 2-Dihydroxymethyl-1-methyl-chinolinium-p-toluolsulfonat
W3 : 1-Hydroxy-3-nitro-4-(3-hydroxypropylamino)-benzol
W4 : 4-(2-Hydroxyethylamino)-3-nitrophenol

| Rezeptur-Nr. | Farbstoff(vorprodukt)e | | | | | | |
|---|---|---|---|---|---|---|---|
| | E1 | K1 | K2 | W1 | W2 | W3 | W4 |
| FZ1 | 0,5 | 0,5 | -- | -- | -- | -- | -- |
| FZ2 | 0,5 | 0,25 | -- | 0,25 | -- | -- | -- |
| FZ3 | 0,5 | 0,25 | -- | -- | 0,25 | -- | -- |
| FZ4 | 0,5 | 0,25 | -- | -- | -- | 0,25 | -- |
| FZ5 | 0,5 | 0,25 | -- | -- | -- | -- | 0,25 |
| FZ6 | 0,5 | -- | 0,5 | -- | -- | -- | -- |
| FZ7 | 0,5 | -- | 0,25 | 0,25 | -- | -- | -- |
| FZ8 | 0,5 | -- | 0,25 | -- | 0,25 | -- | -- |
| FZ9 | 0,5 | -- | 0,25 | -- | -- | 0,25 | -- |
| FZ10 | 0,5 | -- | 0,25 | -- | -- | -- | 0,25 |

*16.1.3 Ausfärbung*

Jeweils 3,7ml der 20 resultierenden Färbegels wurden mit jeweils 3,9ml einer 6 Gew.-%igen wässrigen Wasserstoffperoxidlösung gemischt. Die resultierenden Anwendungszubereitungen wurden jeweils auf ein Baumwollläppchen (Isobaumwolle, 2 x 2 cm) aufgebracht und nach einer Einwirkzeit bei Raumtemperatur von 30 Minuten mit Wasser gründlich abgespült. Für jede der Kombinationen aus Gelgrundlage und Farbstoff(vorprodukt)en wurden 5 derartige Ausfärbungen durchgeführt, die als Basis für die unter Punkt 16.2 beschriebenen Echtheitsprüfungen dienten.

16.2 Echtheitsprüfungen

*16.2.1 Bestimmung der Lichtechtheit*

Die Baumwollläppchen wurden in eine Metallgestell mit Schablone eingespannt und 72h mit UV-Licht bestrahlt.

*16.2.2 Bestimmung der Dauerwellechtheit*

Die Baumwollläppchen wurden mit 35ml einer kommerziell erhältlichen Poly-Lock-Wellemulsion vollständig benetzt und für 20 Minuten bei 27°C gelagert. Anschließend wurden die Stofflappen mit lauwarmen Wasser gespült, mit 35ml einer kommerziell erhältlichen Poly-Lock-Fixierlösung benetzt und für 10 Minuten bei 27°C gelagert. Abschließend wurden die Stofflappen erneut mit lauwarmen Wasser gespült und getrocknet.

*16.2.3 Bestimmung der Schweißechtheit*

Es wurden 100g einer synthetischen, wässrigen Schweißlösung aus 10,0g Natriumchlorid, 1,0g Dinatriumhydrogenphosphat und 0,25g Histidinhydrochlorid hergestellt, deren pH-Wert mit Zitronensäure auf 3,2 eingestellt wurde.

Die Baumwollläppchen wurden mit jeweils 35ml dieser Lösung vollkommen benetzt und 7 Stunden bei 45°C gelagert. Anschließend werden die Stofflappen mit lauwarmen Wasser ausgespült und getrocknet.

*16.2.4 Bestimmung der Waschechtheit*

Die Baumwollläppchen wurden in einem Ultraschallbad mit einer 1 Gew.-%igen wässrigen Texapon® NSO UP Lösung 30 Minuten lang behandelt. Dies entspricht in etwa einer 12-maligen manuellen Haarwäsche. Anschließend wurden die Stofflappen unter fließendem lauwarmen Wasser ausgewaschen und getrocknet.

16.3 Messergebnisse

*16.3.1 Messmethoden*

Die Färbung der jeweils 5 analog ausgefärbten Baumwollläppchen wurden nach den abgeschlossenen unterschiedlichen Behandlungen unmittelbar nacheinander farbmetrisch an jeweils 4 Messpunkten mit einem Gerät vom Typ Spectra Scan PR 650 vermessen, und die Messergebnisse in den folgenden Tabellen (Punkt 16.3.2 beziehungsweise 16.3.3) zusammengefasst.

Zusätzlich wurde gemäß Formel (I) jeweils der ΔE-Wert des CIELAB-Farbsystems im Bezug zur Färbung eines identisch ausgefärbten Läppchens, welches nach der Ausfärbung nicht weiter behandelt wurde, ermittelt

$$\Delta E=\sqrt{(\Delta L)^2 + (\Delta A)^2 + (\Delta B)^2} \qquad (I)$$

*16.3.2 Echtheitseigenschaften der Vergleichsrezepturen*

| Färbecreme | Echtheitsüberprüfung | L | a | b | dE |
|---|---|---|---|---|---|
| VG + FZ1 | UV-Echtheit | 68,30 | 9,82 | 13,74 | 42,2 |
| | Dauerwellechtheit | 65,12 | 16,90 | 15,59 | 34,6 |
| | Schweißechtheit | 68,63 | 12,22 | 10,75 | 42,0 |
| | Waschechtheit | 52,32 | 27,40 | 22,67 | 17,0 |
| | unbehandelt | 43,87 | 39,85 | 30,46 | -- |
| VG + FZ2 | UV-Echtheit | 66,17 | 10,67 | 14,18 | 36,2 |
| | Dauerwellechtheit | 69,27 | 8,19 | 6,61 | 43,0 |
| | Schweißechtheit | 67,74 | 11,55 | 10,30 | 38,2 |
| | Waschechtheit | 51,08 | 27,91 | 22,49 | 11,9 |
| | unbehandelt | 43,21 | 35,42 | 27,21 | -- |
| VG + FZ3 | UV-Echtheit | 65,38 | 10,98 | 16,13 | 37,1 |
| | Dauerwellechtheit | 71,73 | 7,11 | 8,97 | 46,4 |
| | Schweißechtheit | 68,05 | 11,78 | 11,74 | 39,8 |
| | Waschechtheit | 52,19 | 30,21 | 24,58 | 12,6 |
| | unbehandelt | 42,90 | 37,83 | 28,34 | -- |
| VG + FZ4 | UV-Echtheit | 60,37 | 19,84 | 15,63 | 29,2 |
| | Dauerwellechtheit | 69,51 | 12,87 | 12,95 | 40,7 |
| | Schweißechtheit | 68,44 | 12,26 | 12,44 | 40,6 |
| | Waschechtheit | 54,25 | 29,85 | 25,27 | 15,2 |
| | unbehandelt | 42,81 | 39,44 | 28,32 | -- |
| VG + FZ5 | UV-Echtheit | 60,75 | 19,29 | 16,39 | 26,8 |
| | Dauerwellechtheit | 69,10 | 13,83 | 14,18 | 36,5 |
| | Schweißechtheit | 67,65 | 11,63 | 11,83 | 38,0 |
| | Waschechtheit | 55,36 | 28,61 | 24,61 | 14,0 |
| | unbehandelt | 45,45 | 37,86 | 28,11 | -- |

(fortgesetzt)

| Färbecreme | Echtheitsüberprüfung | L | a | b | dE |
|---|---|---|---|---|---|
| VG + FZ6 | UV-Echtheit | 60,91 | 12,84 | 15,46 | 42,9 |
| | Dauerwellechtheit | 64,70 | 22,96 | 18,36 | 37,0 |
| | Schweißechtheit | 57,43 | 24,03 | 13,18 | 34,8 |
| | Waschechtheit | 53,93 | 30,01 | 24,60 | 22,9 |
| | Unbehandelt | 39,87 | 44,80 | 34,96 | -- |
| VG + FZ7 | UV-Echtheit | 62,45 | 12,95 | 15,66 | 40,4 |
| | Dauerwellechtheit | 69,85 | 11,55 | 7,62 | 49,5 |
| | Schweißechtheit | 57,96 | 23,78 | 12,88 | 32,3 |
| | Waschechtheit | 53,09 | 29,73 | 23,21 | 20,4 |
| | Unbehandelt | 38,93 | 41,37 | 32,13 | -- |
| VG + FZ8 | UV-Echtheit | 64,14 | 12,77 | 17,33 | 40,9 |
| | Dauerwellechtheit | 72,72 | 8,05 | 8,66 | 52,9 |
| | Schweißechtheit | 58,90 | 22,48 | 13,02 | 33,5 |
| | Waschechtheit | 55,27 | 29,05 | 22,98 | 22,1 |
| | Unbehandelt | 40,70 | 42,66 | 32,60 | -- |
| VG + FZ9 | UV-Echtheit | 57,91 | 21,85 | 16,56 | 30,7 |
| | Dauerwellechtheit | 71,17 | 11,02 | 10,20 | 48,6 |
| | Schweißechtheit | 60,02 | 22,38 | 12,92 | 33,3 |
| | Waschechtheit | 54,49 | 32,07 | 25,75 | 18,0 |
| | Unbehandelt | 41,76 | 43,78 | 30,83 | -- |
| VG + FZ10 | UV-Echtheit | 58,02 | 22,79 | 17,90 | 26,8 |
| | Dauerwellechtheit | 67,91 | 16,63 | 14,44 | 36,5 |
| | Schweißechtheit | 59,80 | 22,71 | 12,98 | 38,0 |
| | Waschechtheit | 56,15 | 31,41 | 26,04 | 14,0 |
| | Unbehandelt | 42,14 | 44,12 | 32,40 | -- |

## Patentansprüche

1. Verwendung von farbstabilisierendem Wirkstoff oder Wirkstoffgemischen zur Verbesserung der Waschechtheit von Färbungen keratinischer Fasern umfassend als Wirkstoff wenigstens ein N-haltiges Silikon, Polystyrolsulfonat und/oder Pyrrolidon.

2. Verwendung nach Anspruch 1, **dadurch gekennzeichnet, dass** das N-haltige Silikon ausgewählt ist aus der Gruppe umfassend Siloxanpolymere mit wenigstens einer Aminogruppe, vorzugsweise Siloxanpolymere mit wenigstens einer endständigen Aminogruppe, Amodimethicon, Trimethylsilylamodimethicone, bevorzugt Aminoethylaminopropylsiloxan-Dimethylsiloxan-Copolymer und/oder besonders bevorzugt Amodimethicon.

3. Verwendung nach Anspruch 1 oder 2, **dadurch gekennzeichnet, dass** das N-halige Silikon die nachstehende allgemeine Formel I aufweist,

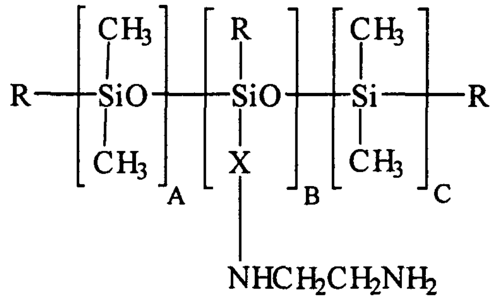

(I)

wobei R = OH oder $CH_3$ ; X = Alkylgruppe mit 1 bis 4 C-Atomen, vorzugsweise Propyl oder Isopropyl, und A, B und C = Copolymereinheiten, die taktische und/oder ataktische Polymerblöcke ausbilden können, sind.

4. Verwendung nach Anspruch 1, **dadurch gekennzeichnet, daß** das Polystyrolsulfonat ein Molekulargewicht zwischen 300 und 1000000 g/mol aufweist.

5. Verwendung nach Anspruch 1, **dadurch gekennzeichnet, daß** das Pyrrolidon die nachstehende allgemeine Formel II aufweist,

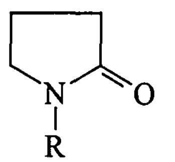

(II)

wobei R = Alkyl, Alkenyl, Hydroxyalkyl, Hydroxyalkenyl, Aminoalkyl, Aminoalkenyl, vorzugsweise mit 8 bis 30 C-Atomen, bevorzugt 8 bis 22 C-Atome, ist.

6. Verwendung nach Anspruch 1 oder 5, **dadurch gekennzeichnet, daß** das Pyrrolidon in Form eines Salzes vorliegt, vorzugsweise quarternisiert mit Dimethylsulfat (DMS) oder als Alkylhalogenid.

7. Verwendung nach einem der vorherigen Ansprüche, **dadurch gekennzeichnet, daß** zusätzlich ein Polymer enthalten ist, wobei das Polymer vorzugsweise ein natürliches oder synthetisches kationisches Polymer ist.

8. Verwendung nach Anspruch 7, **dadurch gekennzeichnet, daß** das kationische Polymer ausgewählt ist aus Homopolymeren der allgemeinen Formel (III),

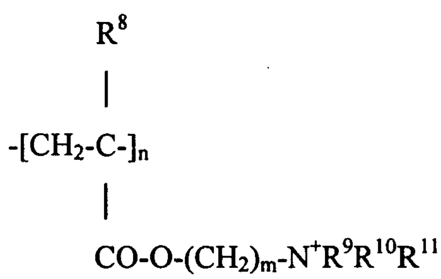

$X^-$ (III)

in der $R^8$ = -H oder -$CH_3$ ist, $R^9$, $R^{10}$ und $R^{11}$ unabhängig voneinander ausgewählt sind aus $C_{1-4}$-Alkyl-, -Alkenyl- oder -Hydroxyalkylgruppen, m = 1, 2, 3 oder 4, n eine natürliche Zahl und $X^-$ ein physiologisch verträgliches organisches oder anorganisches Anion ist, und Copolymeren, bestehend im wesentlichen aus den in Formel (III) aufgeführten Monomereinheiten sowie nichtionogenen Monomereinheiten.

**9.** Verwendung nach Anspruch 7, **dadurch gekennzeichnet, daß** das Polymer Chitosan oder ein Derivat des Chitosanes ist.

**10.** Verwendung nach Anspruch 7, **dadurch gekennzeichnet, daß** die kationischen Polymere ausgewählt sind aus der Gruppe, die

- kationisierte Cellulose-Derivate,
- kationisierte Guar-Derivate,
- kationisierten Honig und/oder
- kationische Alkylpolyglycoside umfaßt.

**11.** Verwendung nach Anspruch 7, **dadurch gekennzeichnet, daß** das zusätzliche Polymer ein anionisches Polymer ist.

**12.** Verwendung nach Anspruch 11, **dadurch gekennzeichnet, daß** das anionische Polymer ein anionisches Polymer ist, das Carboxylat- und/oder Sulfonatgruppen aufweist.

**13.** Verwendung nach Anspruch 11, **dadurch gekennzeichnet, daß** das anionische Polymer als Monomer 2-Acrylamido-2-methylpropansulfonsäure enthält, wobei die Sulfonsäuregruppe ganz oder teilweise als Natrium-, Kalium-, Ammonium-, Mono- oder Triethanolammonium-Salz vorliegen kann.

**14.** Verwendung nach Anspruch 11, **dadurch gekennzeichnet, daß** das anionische Polymer ein Copolymer aus mindestens einem anionischen Monomer und mindestens einem nichtionogenen Monomer ist.

**15.** Verwendung nach Anspruch 11, **dadurch gekennzeichnet, daß** das anionische Polymer ein Copolymer ist, das besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt.

**16.** Verwendung nach einem der vorherigen Ansprüche, **dadurch gekennzeichnet, daß** der Wirkstoff in Kombination mit einem Proteinhydrolysat oder einem Derivat eines Proteinhydrolysats eingesetzt wird.

**17.** Verwendung nach Anspruch 16, **dadurch gekennzeichnet, daß** das Proteinhydrolysat oder Derivat des Proteinhydrolysats pflanzlichen Ursprungs ist.

**18.** Verwendung nach einem der vorherigen Ansprüche, **dadurch gekennzeichnet, daß** der Wirkstoff in Kombination mit einem kationischen Tensid eingesetzt wird.

**19.** Verwendung nach einem der vorherigen Ansprüche, **dadurch gekennzeichnet, daß** der Wirkstoff in Kombination mit einem Vitamin, einem Provitamin, einer Vitaminvorstufe oder einem Derivat davon eingesetzt wird.

**20.** Verwendung nach einem der vorherigen Ansprüche, **dadurch gekennzeichnet, daß** der Wirkstoff in Kombination mit einem Pflanzenextrakt eingesetzt wird.

**21.** Mittel zur Verbesserung der Waschechtheit gefärbter Fasern, insbesondere keratinischer Fasern, gemäß einem der vorherigen Ansprüche.

**22.** Verfahren zur Färbung von Fasern, insbesondere keratinischer Fasern, **dadurch gekennzeichnet, daß** in einem ersten Schritt die Fasern in üblicher Weise gefärbt werden und in einem zweiten Schritt ein Mittel auf die Fasern aufgetragen wird, das mindestens den Wirkstoff oder eine Wirkstoffkombination gemäß einem der Ansprüche 1 bis 20 zur Erhöhung der Waschechtheit der Färbung enthält, wobei das Mittel gegebenenfalls nach einer Einwirkzeit von 1 bis 5 Minuten wieder ausgespült wird.

**23.** Verfahren zur Färbung von Fasern, insbesondere keratinischer Fasern, **dadurch gekennzeichnet, daß** in einem ersten Schritt ein Mittel auf die Fasern aufgetragen wird, das mindestens den Wirkstoff oder eine Wirkstoffkombination gemäß einem der Ansprüche 1 bis 20 zur Erhöhung der Waschechtheit der Färbung enthält, und anschließend in einem zweiten Schritt die keratinischen Fasern in üblicher Weise gefärbt werden.

**24.** Mittel enthaltend einen Wirkstoff und/oder eine Wirkstoffkombination gemäß Anspruch 1 und ein Kohlenhydrat.